**Europäisches Patentamt**

**European Patent Office**

**Office européen des brevets**

(19)

(11) Veröffentlichungsnummer: **0 254 918**
**B1**

## EUROPÄISCHE PATENTSCHRIFT

(12)

(45) Veröffentlichungstag der Patentschrift:
**19.12.90**

(51) Int. Cl.⁵: **A61F 5/56**

(21) Anmeldenummer: **87109770.5**

(22) Anmeldetag: **07.07.87**

(54) Vorrichtung zum Verhindern des menschlichen Schnarchens.

(30) Priorität: **30.07.86 DE 3625790**

(43) Veröffentlichungstag der Anmeldung:
**03.02.88 Patentblatt 88/5**

(45) Bekanntmachung des Hinweises auf die Patenterteilung:
**19.12.90 Patentblatt 90/51**

(84) Benannte Vertragsstaaten:
**AT BE CH DE ES FR GB GR IT LI LU NL SE**

(56) Entgegenhaltungen:
**US-A- 4 114 614**
**US-A- 4 304 227**

(73) Patentinhaber: **Qualmann, Horst,**
**Theodor-Storm-Weg 17, D-2090 Winsen/Luhe(DE)**

(72) Erfinder: **Qualmann, Horst, Theodor-Storm-Weg 17,**
**D-2090 Winsen/Luhe(DE)**

(74) Vertreter: **Schöning, Hans-Werner, Dipl.-Ing. et al,**
**Patentanwälte Niedmers & Schöning Jessenstrasse 4,**
**D-2000 Hamburg 50(DE)**

ACTORUM AG

## Beschreibung

Die Erfindung betrifft eine Vorrichtung zum Verhindern des menschlichen Schnarchens.

Die sehr störende Wirkung des Schnarchens eines Menschen auf seine Mitmenschen ist hinlänglich bekannt. Es hat vielfältige Versuche gegeben, das Schnarchen eines Menschen mit den verschiedensten therapeutischen Mitteln zu beheben, beispielsweise durch medikamentöse Behandlung, durch mechanische Mittel, mit denen beispielsweise das Herabsinken des Kiefers während des Schlafens verhindert wurde, oder auch durch Hypnose. Alle diese bekannten Mittel haben den Nachteil, daß sie erhebliche Nebenwirkungen auf das allgemeine Wohlbefinden des damit behandelten Menschen ausüben, wobei insbesondere die medikamentöse Behandlung diese Probleme in der Regel nicht zum gewünschten Erfolg führt. Bekannte mechanische Vorrichtungen, die beispielsweise von außen an den Kiefer angelegt werden, üben eine erhebliche Beeinträchtigung aus, da sie selbst nur manuell lösbar sind, was zu erheblichen Zuständen der Beklemmung führen kann, wenn die Person beispielsweise unverhofft aufwacht.

Es ist Aufgabe der vorliegenden Erfindung, eine Vorrichtung zum Verhindern des menschlichen Schnarchens zu schaffen, die bei der die Vorrichtung tragende Person keine Gefühle der Beklemmung verursacht, die zudem leicht außer Funktion gebracht werden kann und den Menschen in seinem Wohlbefinden nicht beeinträchtigt, die darüber hinaus auf wirkungsvolle Weise das Schnarchen unterbindet und schließlich auch unter dem Gesichtspunkt der Mundhygiene völlig unbedenklich ist.

Gelöst wird die Aufgabe gemäß der Erfindung durch ein jeweils über den Oberkiefer und den Unterkiefer des Mundes stülpbares und an diese im wesentlichen formmäßig angepaßtes Haubenpaar, wobei die eine Haube permanentmagnetische Eigenschaften und die andere Haube ferromagnetische Eigenschaften aufweist.

Der Vorteil dieser Vorrichtung liegt darin, daß diese gebißartig lediglich über den Oberkiefer und den Unterkiefer gestülpt zu werden braucht, wobei die eine permanentmagnetische Haube des Haubenpaares in bezug auf die permanentmagnetische Kraft so eingestellt ist, daß einerseits bei entspannten Kiefermuskeln beide Kiefer dennoch durch den Permanentmagneten aneinandergehalten werden, andererseits aber dennoch ein Öffnen der Kiefer ohne weiteres möglich ist, wenn die Muskeln zum Öffnen des Kiefers betätigt werden.

Ein Eingeschnürtsein, wie bei den bekannten mechanischen, von außen an den Kiefern angelegten Vorrichtungen, tritt hier nicht auf, da der Benutzer der Vorrichtung diese ohne weiteres außer Eingriff bringen kann, indem er entgegen der Kraftwirkung des Permanentmagneten die Kiefer voneinander entfernt.

Grundsätzlich kann die Haube mit den permanentmagnetischen Eigenschaften als auch die Haube mit den ferromagnetischen Eigenschaften, die als Haubenpaar zum Zusammenhalten der Kiefer miteinander zusammenwirken, grundsätzlich auf beliebige geeignete Weise ausgebildet sein. Vorteilhafterweise sind jedoch die Hauben an ihren mit den Backenzähnen korrespondierenden Bereichen den Kauflächen der Zähne entsprechend flach ausgebildet und weisen an ihren mit den Schneidezähnen korrespondierenden Bereichen eine gegenüber den Bereichen der Kaufläche verminderte Breite auf. Bei entsprechend dünnwandig ausgebildeten Hauben spürt somit der Benutzer einer solchen Vorrichtung die einzelnen Haubenpaare nahezu überhaupt nicht mehr, da diese dadurch dem Kiefer (Oberkiefer, Unterkiefer), insbesondere in bezug auf seine zu den Schneidezähnen hin schmal ausgebildete Formation und zu den Backenzähnen hin breit ausgebildete Formation, angepaßt sind.

Auch der das Haubenpaar jeweils bildende Werkstoff kann grundsätzlich ein beliebiger geeigneter Werkstoff sein. Gemäß einer vorteilhaften Ausführungsform der Erfindung ist das Haubenpaar jedoch in individueller Anpassung an den Kiefer des Trägers der Vorrichtung aus Kunststoff herstellbar. Vorzugsweise ist der Kunststoff dabei ein solcher, wie er für die Aufnahme von Ersatzzähnen in Totalprothesen verwendet wird.

Um zu verhindern, daß sich die Hauben selbst während des Schlafens vom Oberkiefer bzw. vom Unterkiefer lösen, die Kiefer sich wieder voneinander entfernen und das Schnarchen wiederum beginnt, sind vorzugsweise die stegartigen Seiten beider Hauben des Paares, die zusammen mit dem unteren querstegartigen Bereichen ein im wesentlichen U-förmiges Querschnittsprofil bilden, derart geneigt, daß die von ihm gebildete Eintrittsöffnung geringfügig kleiner als die Breite der querstegartigen Bereiche ist. Durch diese Art der Ausbildung können diese, indem die Stege beim Aufstülpen auf die Zähne bzw. Kiefer leicht voneinander entfernt werden, mit geringfügiger Spannung auf den Zähnen bzw. auf dem Kiefer gehalten werden. Zusätzlich können die Hauben, wenn sie aufgrund der jeweiligen Ausbildung des Kiefers bzw. der Zähne dennoch nicht in allen Fällen gesichert gehalten werden können, zusätzlich mit bekannten flüssigen Haftmitteln, wie sie auch für Totalprothesen verwendet werden, am Kiefer bzw. an den Zähnen fixiert werden.

Die ferromagnetische Eigenschaft der einen Haube und die permanentmagnetische Eigenschaft der anderen Haube kann wiederum durch beliebige geeignete Maßnahmen bewirkt werden. Vorteilhafterweise weist jedoch die Haube, die die ferromagnetische Eigenschaft aufweisen soll, in sie eingelagerte ferromagnetische Fäden und/oder Plättchen auf, die auf geeignete Weise über die gesamte Ausdehnung der jeweiligen Hauben in bestimmten Abständen zueinander angeordnet sind. Es ist aber auch denkbar, beispielsweise die ferromagnetischen bzw. permanentmagnetischen Fäden durchgehend über die jeweilige gesamte Haube auszubilden. Darüber hinaus sind auch Kombinationen möglich, beispielsweise bei denen der einen Haube ferromagnetische Fäden und in der anderen, ihr gegenüberliegenden Haube, permanentmagnetische Plättchen oder umgekehrt angeordnet sind.

Schließlich ist es auch denkbar, die ferromagnetischen und permanentmagnetischen Mittel in Form einer Beschichtung entweder unmittelbar auf den gegenüberliegenden Oberflächen der Hauben auszubilden, oder diese unterhalb der gegenüberliegenden Fläche in den Werkstoff der Hauben einzulagern.

Gemäß einer vorteilhaften weiteren Ausführungsform der Vorrichtung können schließlich die Fäden und/oder die Plättchen der ferromagnetischen und der permanentmagnetischen Haube aus diese Eigenschaften aufweisendem Metall bestehen, grundsätzlich können aber auch die Fäden, die Plättchen oder auch die ebenso geeigneten Beschichtungen aus künstlichen Werkstoffen hergestellt sein, die permanentmagnetische bzw. ferromagnetische Eigenschaften aufweisen.

Die Erfindung wird nun unter Bezugnahme auf die nachfolgenden schematischen Zeichnungen anhand eines Ausführungsbeispiels eingehend beschrieben.

Darin zeigen:

Fig. 1 eine Draufsicht von unten auf ein über den Oberkiefer und Unterkiefer eines Menschens gestülptes Haubenpaar bei weit geöffnetem Kiefer,

Fig. 2 in der Seitenansicht im Schnitt ein auf einanderliegendes Haubenpaar bei geschlossenem Kiefer,

Fig. 3 ein auf die Kiefer gestülptes Haubenpaar bei geschlossenem Kiefer in der Ansicht von vorn im Schnitt und

Fig. 4 einen Schnitt durch eine Haube entlang der Linie A-B von Fig. 2 bei weggelassenem Kiefer in entspanntem Zustand.

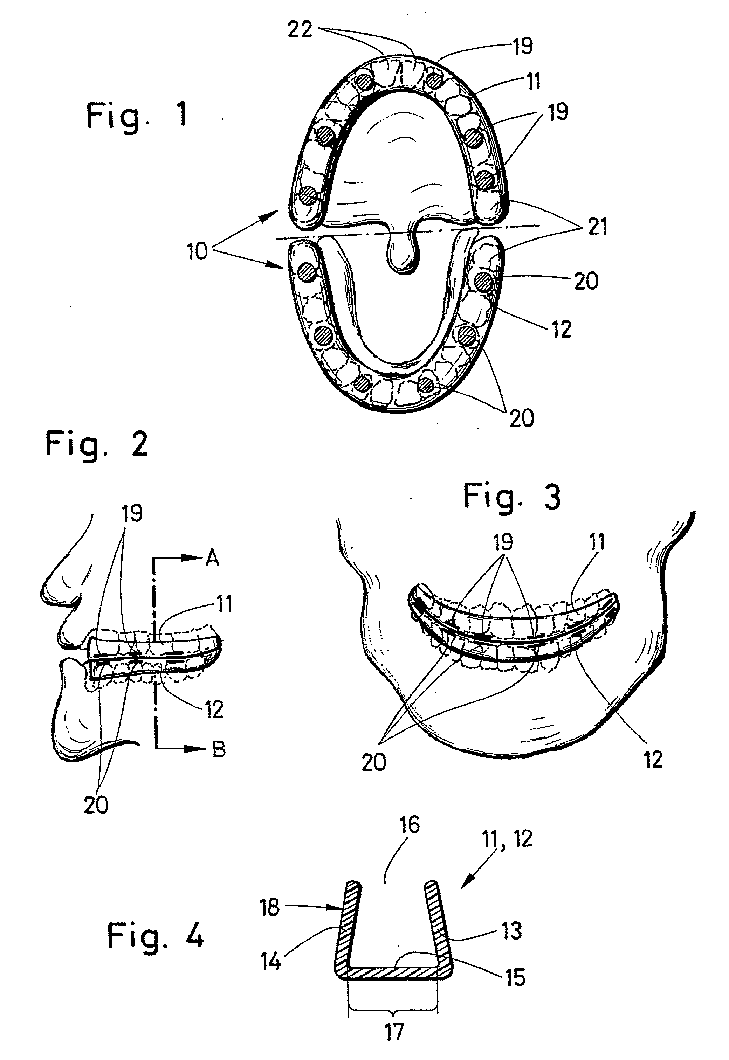

Die Vorrichtung 10 besteht im wesentlichen aus einem Haubenpaar 11, 12, das eine im wesentlich U-förmige Querschnittsform 18 (vgl. Fig. 4) aufweist. Das Haubenpaar, d.h. die obere Haube 11 und die untere Haube 12 sind dabei an ihren mit Backenzähnen 21 korrespondierenden Bereichen den Kauflächen der dortigen Zähne entsprechend flach ausgebildet und weisen an ihren mit den Schneidezähnen 22 korrespondierenden Bereichen eine gegenüber den Bereichen der Kaufläche verminderte Breite auf.

Im Querschnitt sind die eine Haube 11 (oben) und die andere Haube 12 (unten) im wesentlichen gleich ausgebildet, d.h. sie weisen zwei stegartige Seiten 13, 14 und einen dazu querstegartigen Bereich 15, der im wesentlichen der Kaufläche der Zähne gegenübersteht, auf. Die beiden stegartigen Seiten 13, 14 der Hauben 11, 12 sind derart zueinander geneigt, daß die zwischen den beiden Seiten 13, 14 gebildete Eintrittsöffnung 16 geringfügig kleiner als die Breite 17 des inneren querstegartigen Bereiches 15 ausgebildet ist. Da der die Hauben 11, 12 bildende Werkstoff flexibel ist, können die beiden Seiten 13, 14 beim Aufschieben der Hauben 11, 12 auf den Ober- bzw. Unterkiefer geringfügig voneinander wegfedern und klemmen dabei den Kiefer bzw. die Zähne zwischen sich ein. Dadurch wird eine sehr gute Anlage der beiden Hauben 11, 12 am jeweiligen Kiefer erreicht.

Im querstegartigen Bereich der einen Haube 11 sind längs seines gesamten Ausdehnungsbereiches in bestimmten Abständen Plättchen 19 angeordnet, die beispielsweise aus einem ferromagnetischem Stoff bestehen. Ebenso sind in der anderen Haube 12 an bestimmten Stellen längs seines querstegartigen Bereiches 15 entsprechend voneinander beabstandete Plättchen 20 angeordnet, die permanentmagnetische Eigenschaften aufweisen. Wie aus den Figuren 2 und 3 ersichtlich ist, sind die Plättchen 19, 20 dabei so angeordnet, daß sie bei geschlossenem Kiefer in etwa gegenüberliegen, so daß die magnetisch wirkende Haube 11 ihre durch den Magnetismus bewirkte Kraft auf die Haube 12 ausüben kann, d.h. sie aneinandergezogen hält.

Anstelle der ferromagnetischen bzw. permanentmagnetischen Plättchen 19, 20 können auch ferromagnetische bzw. permanentmagnetische Fäden in den jeweiligen querschnittartigen Bereichen 15 der Hauben 11, 12 vorgesehen sein, wobei auch Kombinationen zwischen Plättchen und Fäden sowohl auf der ferromagnetisch wirkenden Haube als auch auf der permanentmagnetischen Haube möglich sind. Darüber hinaus können auch Beschichtungen auf dem querschnittartigen Bereich 15 der jeweiligen Hauben 11, 12 vorgesehen sein, wobei die Plättchen 19, 20, die Fäden, oder auch die ferromagnetische bzw. permanentmagnetische Beschichtung sowohl auf der Oberflächen der Hauben 11, 12 angeordnet sein können als auch unmittelbar unterhalb der Oberfläche des querstegartigen Bereiches 15. Im letzteren Falle wären die ferromagnetischen bzw. permanentmagnetischenMittel nicht sichtbar und nicht fühlbar, was wiederum den Vorteil hat, daß die Vorrichtung 10 auch als absolut geschmacksneutral empfunden werden kann, insbesondere, wenn der die Hauben 11, 12 bildende Werkstoff ein Kunststoff ist, wie er als Träger bzw. Aufnahme von Ersatzzähnen in Totalprothesen verwendet wird.

Die Vorrichtung 10 selbt läßt sich grundsätzlich auf einfache Weise herstellen und kann individuell an den Kiefer des Benutzers angepaßt werden. Dazu wird zunächst ein Abdruck des Kiefers auf allgemein bekannte Art abgenommen. Dann wird in einem weiteren Herstellungsvorgang ein Gibsabdruck des Ober- und Unterkiefers angefertigt. Auf der Grundlage dieses Gibsabdruckes werden dann die Hauben individuell angefertigt, und zwar nach Maßgabe der vorangehend geschilderten konstruktiven Einzelheiten.

Bei der Benutzung wird dann das Haubenpaar jeweils auf den Ober- bzw. Unterkiefer aufgesetzt und der Kiefer geschlossen. Das Öffnen des Mundes des Benutzers im Schlaf wird durch die magnetisch bedingte Kraftwirkung zwischen beiden Hauben 11, 12 unterbunden, wobei die schlafende Person dadurch gehalten wird, durch die Nase zu atmen. Vorteilhaft ist weiterhin, daß die Vorrichtung 10 nach dem Aufsetzen auf die Zähne bzw. die Kiefer von außen nicht sichtbar ist, d.h. die ästhetische Empfindung nicht gestört wird.

Falls es während des Schlafens doch infolge einer eventuell auftretenden Atemnot erforderlich sein sollte, den Mund schnell zu öffnen, kann der Benutzer durch leicht erhöhte Kraft - im Sinne einer Öffnung des Mundes - beide Hauben 11, 12 voneinander trennen, da die magnetische Kraftwirkung bei der Herstellung so eingestellt worden ist, daß dafür kein hoher Kraftaufwand nötig ist.

Schließlich ist noch hervorzuheben, daß aufgrund des totalen Eingebettetseins der Mittel, die in der einen Haube die permanentmagnetischen Eigenschaften bewirken und in der anderen Haube die ferromagnetischen Eigenschaften, daß die Vorrichtung 10 insgesamt auch unter hygienischen Gesichtspunkten bedenkenlos benutzt werden kann, zumal dann, wenn zur Ausbildung der Hauben 11, 12 ein Kunststoff verwendet wird, wie er normalerweise für Totalprothesen Verwendung findet. Auch ist eine Reinigung der Vorrichtung 10 bei Verwendung dieses Kunstwerkstoffes unproblematisch.

Bezugszeichenliste

10 Vorrichtung
11 Haube (oben)
12 Haube (unten)
13 stegartige Seite
14 stegartige Seite
15 querstegartiger Bereich
16 Eintrittsöffnung
17 Breite des querstegartigen Bereiches
18 U-förmiges Querschnittsprofil
19 ferromagnetischer Faden/Plättchen
20 permanentmagnetischer Faden/Plättchen
21 Backenzahn
22 Schneidezahn

**Patentansprüche**

1. Vorrichtung zum Verhindern des menschlichen Schnarchens, gekennzeichnet durch ein jeweils über den Oberkiefer und den Unterkiefer des Menschen stülpbares und an diese im wesentlichen formmäßig angepaßtes Haubenpaar (11, 12), wobei die eine Haube (11) permanent-magnetische Eigenschaften und die andere Haube (12) ferromagnetische Eigenschaften aufweist.

2. Vorrichtung nach Anspruch 1, dadurch gekennzeichnet, daß die Hauben (11, 12) an ihrem mit den Backenzähnen korrespondierenden Bereichen den Kauflächen der Zähne entsprechend flach ausgebildet sind und an ihren mit den Schneidzähnen korrespondierenden Bereichen eine gegenüber den Bereichen der Kaufläche verminderte Breite aufweisen.

3. Vorrichtung nach einem oder beiden der Ansprüche 1 oder 2, dadurch gekennzeichnet, daß das Haubenpaar (11, 12) in individueller Anpassung an den Kiefer des Trägers der Vorrichtung (10) aus Kunststoff herstellbar ist.

4. Vorrichtung nach Anspruch 3, dadurch gekennzeichnet, daß der Kunststoff ein als Träger von Ersatzzähnen in Totalprothesen dienender Kunststoff ist.

5. Vorrichtung nach einem oder mehreren der Ansprüche 1 bis 4, dadurch gekennzeichnet, daß die stegartigen Seiten (13, 14) beider Hauben (11, 12) des Paares, die zusammen mit den unteren querstegartigen Bereichen (15) ein im wesentlichen U-förmiges Querschnittsprofil (18) bilden, derart geneigt sind, daß die von ihm gebildete Eintrittsöffnung (16) geringfügig kleiner als die Breite (17) der querstegartigen Bereiche (15) ist.

6. Vorrichtung nach einem oder mehreren der Ansprüche 1 bis 5, dadurch gekennzeichnet, daß die Hauben (11, 12) in sie eingelagerte ferromagnetische Fäden und/oder Plättchen (19) aufweisen.

7. Vorrichtung nach einem oder mehreren der Ansprüche 1 bis 6, dadurch gekennzeichnet, daß die Hauben (11, 12) in sie eingelagerte permanentmagnetische Fäden und/oder Plättchen(20) aufweisen.

8. Vorrichtung nach einem oder beiden der Ansprüche 6 oder 7, dadurch gekennzeichnet, daß die Fäden und/oder Plättchen (19; 20) aus Metall bestehen.

**Claims**

1. A device for preventing human snoring, characterised by a pair of caps (11, 12) substantially matching the shape of and adapted to be fitted over the upper jaw and the lower jaw of the person, one cap (11) having permanent magnetic properties while the other cap (12) has ferromagnetic properties.

2. A device according to claim 1, characterised in that the portions of the caps (11, 12) which correspond to the molar teeth are constructed flat in accordance with the masticating surfaces of the teeth, while their portions which correspond to the incisors have a width which is reduced in relation to the areas of the mastication surface.

3. A device according to one or both of claims 1 or 2, characterised in that the pair of caps (11, 12) can be produced from synthetic plastics material and individually adapted to the jaws of the wearer of the device (10).

4. A device according to claim 3, characterised in that the synthetic plastics material is a synthetic plastics material which is used as a carrier for permanent teeth in total prostheses.

5. A device according to one or more of claims 1 to 4, characterised in that the web-like sides (13, 14) of the two caps (11, 12) of the pair, which together with the lower transverse web-like zones (15), form a substantially U-shaped cross-sectional profile (18), are so inclined that the entrance aperture (16) formed by it is slightly smaller than the width (17) of the transverse web-like zones (15).

6. A device according to one or more of claims 1 to 5, characterised in that the caps (11, 12) comprise tiny ferromagnetic plates (19) and/or threads incorporated into them.

7. A device according to one or more of claims 1 to 6, characterised in that the caps (11, 12) have tiny permanent magnetic plates (20) and/or threads incorporated into them.

8. A device according to one or both of claims 6 or 7, characterised in that the tiny plates and/or threads (19, 20) consist of metal.

**Revendications**

1. Dispositif pour éviter le ronflement humain, caractérisé par une paire de bonnets (11, 12) applicables sur la mâchoire supérieure et la mâchoire inférieure de l'homme, et adapté sensiblement à celles-ci, l'un des bonnets (11) possédant des propriétés magnétiques permannetes et l'autre bonnet (12) des propriétés ferromagnétiques.

2. Dispositif selon la revendication 1, caractérisé en ce que les bonnets (11, 12), dans leur zone correspondant aux molaires sont d'une structure plate correspondant aux surfaces de mastiquage des dents, et, dans leurs zones correspondant aux incisives, présentent une largeur réduite par rapport aux zones de la surface de mastiquage.

3. Dispositif selon l'une des revendications 1 ou 2, caractérisé en ce que la paire de bonnets (11, 12) peut être fabriquée en matière plastique en s'adaptant individuellement à la mâchoire du porteur du dispositif (10).

4. Dispositif selon la revendication 3, caractérisé en ce que la matière plastique est une matière plastique servant de support de dentiers dans des prothèses totales.

5. Dispsitif selon l'une ou plusieurs des revendications 1 à 4, caractérisé en ce que les côtés en forme d'entretoises (13, 14) des deux bonnets (11, 12) de la paire, qui constituent, avec les zones (15) inférieures en forme d'entretoises transversales un profil transversal sensiblement en forme de U (18) sont inclinés de telle façon que l'orifice d'entrée (16) constitué par ce profil est légèrement plus petite que la largeur (17) des zones (15) formant entretoise transversales.

6. Dispositif selon l'une ou plusieurs des revendications 1 à 5, caractérisé en ce que les bonnets (11, 12) présentent des fils et/ou des plaquettes (19) ferromagnétiques insérés.

7. Dispositif selon l'une ou plusieurs des revendications 1 à 6, caractérisé en ce que les bonnets (11, 12) présentent des fils et/ou des plaquettes (20) à aimant permanents qui y sont insérés.

8. Dispositif selon l'une des revendications 6 ou 7 ou selon les deux revendications, caractérisé en ce que les fils et/ou les plaquettes (19, 20) sont en métal.

Fig. 1

Fig. 2

Fig. 3

Fig. 4